# EUROPEAN PATENT SPECIFICATION

(11) **EP 0 477 020 B1**
(45) Date of publication and mention of the grant of the patent: **22.11.1995**
(21) Application number: 91308566.8
(22) Date of filing: 19.09.1991
(51) Int. Cl.: A61B 17/12, A61L 17/00

(54) **Polymeric endoscopic ligature**
Polymerische endoskopische Abbindung
Ligature endoscopique polymérique

(30) Priority: 20.09.1990 US 585757
(43) Date of publication of application: 25.03.1992
(73) Proprietor: ETHICON INC., Somerville New Jersey 08876 (US)
(72) Inventor: Chen, Chao, Edison, New Jersey 08820 (US); Weaver, Gregory, New Hope, Pennsylvania 18938 (US); Kindberg, Richard, Doylestown, Pennsylvania 18901 (US); Carroll, Kevin, Phillipsburg New Jersey 08865 (US); Spengler, Ralph, Long Valley, New Jersey 07853 (US)
(74) Representative: Fisher, Adrian John

(56) References cited:
- EP-A- 0 132 131
- WO-A-86/00020
- WO-A-90/06725
- DE-A- 2 900 265
- FR-A- 736 756
- GB-A- 2 033 411
- US-A- 1 856 721
- US-A- 3 476 115
- US-A- 4 300 565
- US-A- 4 735 194
- US-A- 4 838 267

## Description

### BACKGROUND OF THE INVENTION

As medical and hospital costs continue to increase, surgeons are constantly striving to develop advanced surgical techniques. Advances in the surgical field are often related to the development of operative techniques which involve less invasive surgical procedures and reduce overall patient trauma. In this manner, the length of hospital stays can be significantly reduced, and therefore the hospital and medical costs can be reduced as well.

One of the truly great advances in recent years to reduce the invasiveness of surgical procedures is endoscopic surgery. Endoscopic surgery involves the use of an endoscope, which is an instrument permitting the visual inspection and magnification of any cavity of the body. The endoscope is inserted through a cannula after puncture through the wall of the body cavity with a trocar, which is a sharp-pointed instrument. The surgeon can then perform diagnostic and therapeutic procedures at the surgical site with the aid of specialized instrumentation designed to fit through additional cannulas providing openings into the desired body cavity as may be required.

In many surgical procedures, including those involving endoscopic surgery, it is often necessary to ligate blood vessels which have been cut within the surgical site. The vessels may then be severed downstream of the ligated portion. The primary reason for ligating the vessels is to maintain the surgical site free of an excess of blood and to reduce blood loss in the patient.

In the past, the surgeon closed blood vessels with conventional ligatures, which are long, relatively straight strands of suture material. The surgeon would manually tie the ligature around the vessel desired to be closed. Unfortunately, this is a very time-consuming process, and one certainly not well suited for endoscopic surgical applications where a surgeon's manual operative techniques within the surgical site are severely restricted.

In more recent years, an endoscopic ligature has been developed especially well adapted for endoscopic surgery. ENDOLOOP™ gut ligature is a device formed from a suture material of surgical catgut. The suture material is formed into a ligature loop at the distal end of the device with a knot which becomes secure after the knot is activated. Although ENDOLOOP™ gut ligature facilitates ligation of vessels through small incisions in bodily cavities, the surgical gut from which it is formed may elicit significant tissue reaction. Additionally, the reproducibility of the physical and biological properties of the surgical gut is difficult because it is derived from natural sources.

In view of the deficiencies of the prior art for preparing a suitable endoscopic ligature exhibiting minimal tissue reaction and outstanding physical and biological properties, such an endoscopic ligature would be highly desired within the medical community.

DE-A-2900265 discloses the features of the preamble of claim 1, the latter describes a medical device comprising an endoscopic ligature. One end of the filament making up the ligature is formed into coils, and the other end is passed through the coils. The end forming the coils is passed back through the coils as well. This knot construction ensures that, when the size of the ligature loop has been reduced, it will not expand and then release the ligated object. The ligature can be formed from the usual surgical suture materials such as catgut, artificial silk, cotton thread, or from other synthetic fiber-forming materials.

The present invention provides a medical device for applying a ligature in an endoscopic surgical procedure, in combination with a ligature, characterized in that the ligature is composed of a monofilament of poly para-dioxanone (PDS) having a weight average molecular weight between 40,000 and 120,000.

Surpisingly, endoscopic ligatures fabricated from such PDS monofilaments exhibit excellent knot security and minimal knot slippage when ligating a vessel during edoscopic surgery. These properties, unlike the properties obtained from the conventional gut endoscopic ligatures, are reproducible.

The medical devices of this invention can be used not only to ligate vessels endoscopically during surgery, but also to perform other desirable surgical techniques when used in combination with other fabricated devices particularly adapted for surgery.

Specific embodiments of the present invention will now be described further, by way of example, with reference to the accompanying drawings, in which:-
Figure 1 is a perspective view of one embodiment of the medical device of the present invention incorporating an endoscopic ligature composed of a PDS monofilament;
Figure 2 is a perspective view similar to Figure 1 wherein the device is in the process of ligating a vessel;
Figure 3 is a side elevational view of the device inserted through a cannula/trocar assembly;
Figure 4 is a partial perspective view of the first step in forming a ligature knot for an endoscopic ligature composed of at least one synthetic polymeric filament;
Figure 5 is a perspective view of a completed knot configuration for an endoscopic ligature composed of at least one synthetic polymeric filament in a preferred embodiment of the invention;
Figure 6 is a perspective view of a knot configuration which is used in the prior art endoscopic ligature composed of surgical gut;
Figure 7 is a perspective view of an alternate embodiment of a knot configuration for an endoscopic ligature particularly suitable for polymeric filaments;
Figure 8 is a perspective view of an endoscopic ligature wherein a button or disc is used to form the ligature loop instead of a knot configuration for securely ligating a vessel.

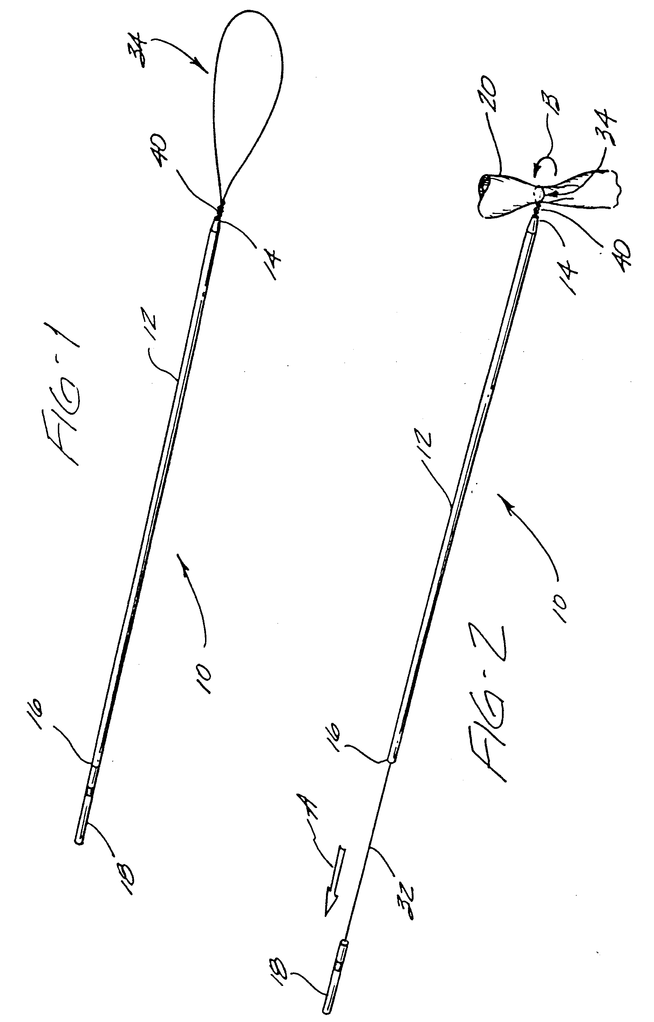

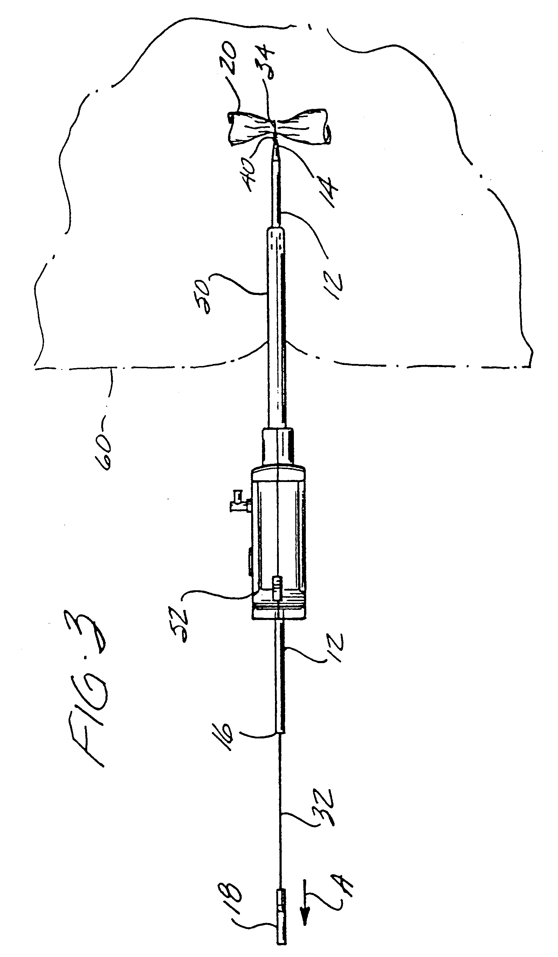

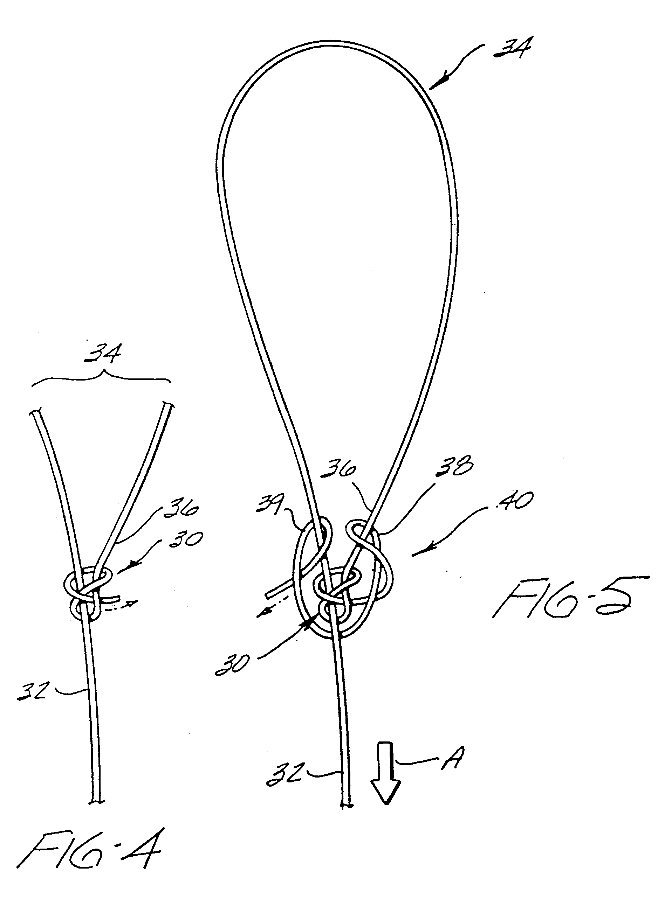

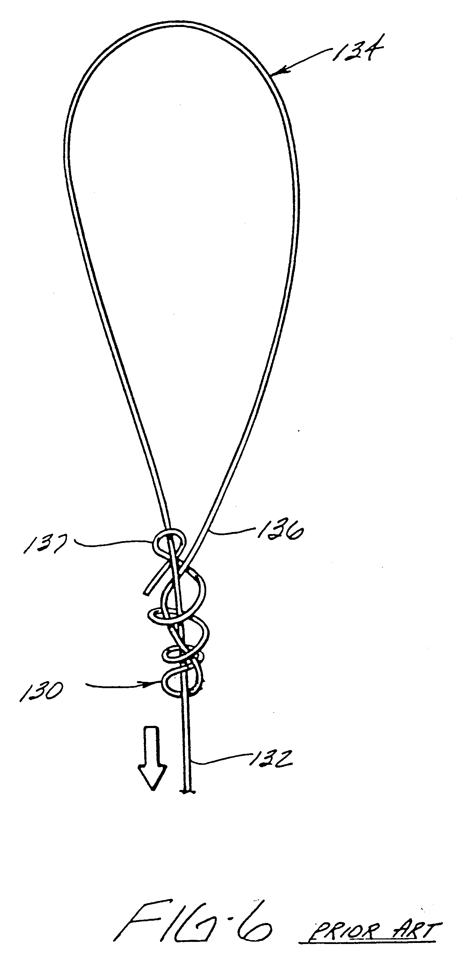

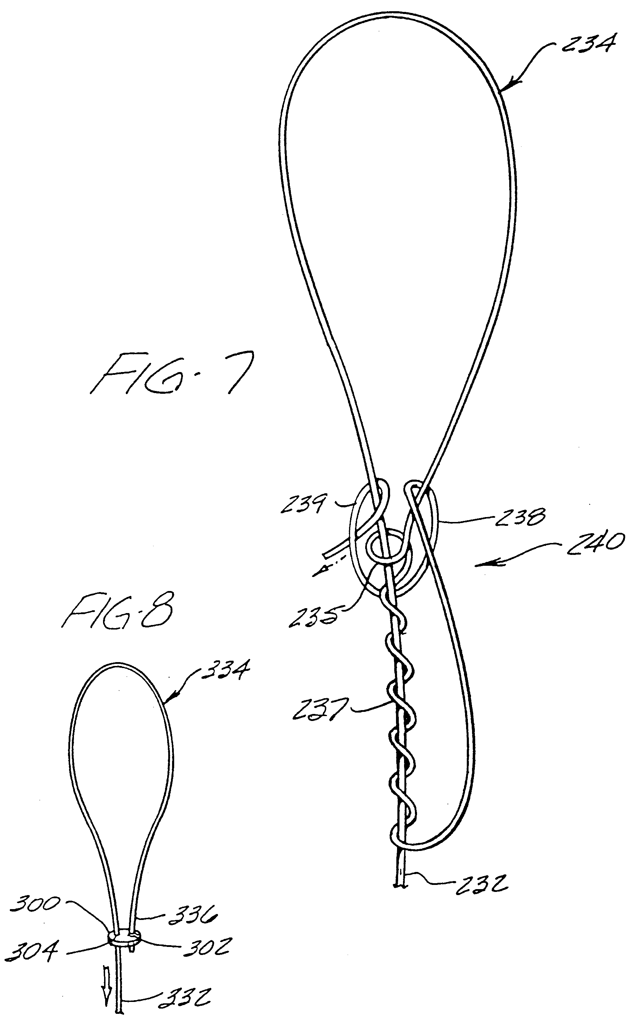

Referring to Figure 1, an embodiment of the medical device of this invention for applying an endoscopic ligature composed of a PDS monofilament is shown generally as 10. The device 10 is comprised of a longitudinal tubular shaft 12, a distal tapered end 14, a score line 16 and a frangible portion 18 located at the proximal end of the device for purposes of gripping. Extending from distal end 14 is an endoscopic ligature which has a loop portion 34 and a knot 40. The endoscopic ligature, as that term is described herein, will be described in greater detail with respect to Figures 4 and 5.

In order to ligate vessel 20, as seen in Figure 2, the user would first grip frangible portion 18 with one hand and the shaft 12 with the other hand and snap apart the two pieces about score line 16. This allows for the continuous filament 32 to be retracted through the hollow shaft 12. Continuous filament 32 is secured to frangible portion 18 by means of adhesive, crimping or any other suitable attaching means. Secondly, the user would then place loop 34 around vessel 20, positioning the ligature at the appropriate point on the vessel 20. To complete the procedure, continuous filament 32 is pulled proximally as shown by arrow "A", causing the loop 34 to ligate vessel 20 as shown by arrow "B". Knot 40 is restrained by distal end 14 and allows continuous filament 32 to pass through to shaft 12 so that the ligature may be tightened securely about vessel 20. A critical feature, one that requires a modified knot configuration for the synthetics, is that the knot must be absolutely "tight" in one direction and should slip in the other. In this manner, once the knot is tightened around the vessel it must remain tight and not loosen.

Referring now to Figure 3, a trocar cannula 50 can be positioned within body cavity 60 to receive medical device 10 for endoscopically ligating vessel 20. Trocar cannula 50 is affixed to main body portion 52 and is hollow throughout its entire length.

Figures 4 and 5 show a method of securing a knot arrangement 40 to prepare an endoscopic ligature composed of at least one synthetic polymeric filament. A basic knot 30 is wrapped about continuous filament 32. This knot 30 is commonly referred to as a #19 knot to those skilled in the art. One would begin forming this knot by laying continuous filament 32 out and forming a loop region 34 which terminates at point 36 adjacent knot 30. The filament is then continued (see Figure 5) to form two additional throws 38 and 39. Once the final knot 40 is completed a sizing gauge is used to properly size loop 34 by adjusting the free end of filament 32. Continuous filament 32 is then passed through medical device 10 and proximal end 18 is secured with epoxy. The additional length of continuous filament 32 is then trimmed off.

Figure 6 shows the prior art endoscopic ligature, which has a basic knot 130, suture material 132 composed of surgical gut, a loop 134 terminating at 136 and a final throw 137 completing the endoscopic ligature. Although this knot configuration exhibits adequate security during ligation with a ligature composed of surgical gut, it is inadequate for securely ligating when a ligature composed of at least one synthetic polymeric filament is used.

Figure 7 depicts another knot configuration for an endoscopic ligature used to prepare the medical device of this invention. To form this configuration one would start by laying continuous filament 232 out and forming a loop 234. Throw 235 is formed and then a spiral wrap 237 continues downward and is brought up to form two throws 238 and 239 completing the knot shown generally as 240. Other knot configurations which exhibit the desired properties may also be possible.

Although the formation of various knot configurations is the preferred means for fabricating the endoscopic ligatures used for preparing medical devices of this invention, such knots are in no way the only way in which endoscopic ligatures composed of a PDS monofilament can be fabricated. For example, figure 8 shows a disc or button as an alternative to the endoscopic ligature knot. Button 300 has an opening 302 in which one end 336 of the filament 332 is secured by epoxy or any other suitable means. Also located on button 300 is an opening 304 for the filament 332 to pass through and form a loop 334. Button 300 acts similar to knots 40 and 240 to restrict the loops 34 and 234 respectively from passing through medical device 10.

The synthetic fiber-forming polymer is fabricated into a monofilament which makes up the endoscopic ligature. Each filament is continuous, so therefore the filament extends substantially along the entire length of the ligature.

The preferred PDS polymers exhibit a weight average molecular weight which render the polymer suitable for extrusion into fibers. Advantageously, the molecular weight of the polymer as measured by gel permeation chromatography ranges from 40,000 to 120,000, preferably from 60,000 to 90,000. A polymer with a molecular weight below 40,000 generally lacks sufficient viscosity to provide suitable melt strength for extrusion, and a polymer with a molecular weight above 120,000 is generally too viscous for melt processing at the temperatures desired to avoid polymer degradation.

The PDS polymer can be fabricated into a filament suitable for the preparation of an endoscopic ligature using conventionally accepted methods well known in the art by first melt extruding the polymer through a spinneret to prepare fibers, drawing the fibers to create orientation, and then annealing the oriented fibers to enhance dimensional stability. Optimum annealing time and temperature for maximum physical and biological properties is readily determined by simple experimentation.

While several embodiments have been depicted, it will be readily apparent to those skilled in the art that numerous modifications in the design or fabrication of the medical device can be made without departing from the scope of this invention as defined in the accompanying claims.

### EXAMPLES

An annealed suture strand of each of the following polymeric filaments is obtained: PDSII monofilament polydioxanone, VICRYL® braided poly(lactide-co-glycolide) (comparative example) ETHILON® monofilament nylon (comparative example), and NUROLON® braided nylon (comparative example). An endoscopic ligature from each of the strands is prepared by first forming a #19 knot (see Fig. 4) and then passing 2 additional throws on each side of the formed loop (see Fig. 5). The final loop size is adjusted by sliding the knot using a loop gauge.

The straight end of the endoscopic ligature is then inserted into a cannula with the knot in contact with the pointed tip of the cannula. Epoxy is injected into the flat end of the cannula to adhere the continuous filamentary strand to the cannula. The excessive length of the strand is trimmed off.

The finished medical device is then placed in a paper folder and foil package. The device is sterilized with ethylene oxide vapor or cobalt radiation, depending on the particular filamentary polymer.

To evaluate the effectiveness of the knot of the endoscopic ligature of the device, the following tests are established:
1) Knot Security Test
   A knot is tightened on a rubber tube containing liquid using 18 N (4 lb.) force. The liquid in the rubber tube is then pressurized to 35 to 69 kPa (5 and 10 psi). The liquid leakage through ligating site is monitored at 1 min., 2 hrs. and overnight intervals. The test is repeated 19 times for separately fabricated devices incorporating endoscopic ligatures composed of the specified fiber-forming polymers.
2) Knot Slippage Test (monofilament only)
   A knot is tightened on a rubber tube pressurized to 45 N (10 lb.) force while the knot is submersed in glycerol. The test is repeated 9 times for separately fabricated devices incorporating endoscopic ligatures composed of the specified fiber-forming polymers.
3) Knotting Angle (braid only)
   While the knot is tightened, the cannula is parallel to the tube. The force required to slide a knot and any liquid leakage at 69 kPa (10 psi) internal pressure are monitored. The test is repeated 9 times for separately fabricated endoscopic ligatures composed of the specified fiber-forming polymers.

The following illustrates the testing results.

| Test | PDSII | VICRYL® | ETHILON® | NUROLON® |
|---|---|---|---|---|
| 1. | 20/20 | 19/20* | 20/20 | 20/20 (no leak) |
| 2. | 10/10 | --- | 10/10 | --- (no slip) |
| 3. | --- | 10/10 | --- | 10/10 (no leak) |

| | | | | |
|---|---|---|---|---|
| *one leak 1 drop/min at 69 kPa (10 psi). | | | | |

The results show that the endoscopic ligatures composed of a PDS monofilament from which the medical devices of this invention are prepared repeatedly demonstrate outstanding knot security and minimal knot slippage for a series of runs.

## Claims

1. A medical device for applying a ligature in an endoscopic surgical procedure, in combination with a ligature characterized, in that the ligature is composed of a monofilament of poly para-dioxanone (PDS) having a weight average molecular weight between 40,000 and 120,000.

2. A medical device according to claim 1, wherein the medical device is in a sterile condition.

## Patentansprüche

1. Medizinische Vorrichtung zum Anlegen eines Ligaturfadens bei einer endoskopischen chirurgischen Maßnahme in Verbindung mit einer Ligatur, dadurch **gekennzeichnet**, daß der Ligaturfaden aus einem Monofilament aus Poly-Para-Dioxanon (PDS) mit einem mittleren Molekulargewicht zwischen 40 000 und 120 000 besteht.

2. Medizinische Vorrichtung nach Anspruch 1, wobei die medizinische Vorrichtung in einem sterilen Zustand ist.

## Revendications

1. Dispositif médical pour appliquer une ligature dans un procédé chirurgical endoscopique en combinaison avec une ligature caractérisé en ce que la ligature est composée d'un monofilament de poly para-dioxanone (PDS) ayant un poids moléculaire moyen en poids compris entre 40 000 et 120 000.

2. Dispositif médical selon la revendication 1, dans lequel le dispositif médical est dans un état stérile.
